# EUROPEAN PATENT APPLICATION

(11) **EP 4 717 261 A1**
(43) Date of publication of application: **01.04.2026**
(21) Application number: 24383033.8
(22) Date of filing: 26.09.2024
(51) Int. Cl.: A61K 9/48, A61K 31/423

(54) **PHARMACEUTICAL COMPOSITIONS OF 2-(3,5-DICHLOROPHENYL)-1,3-BENZOXAZOLE-6-CARBOXYLIC ACID**

(71) Applicant: Galenicum Health S.L.U., 08950 Esplugues de Llobregat (ES)
(72) Inventor: Gómez Coello, Luis, 08950 Esplugues de Llobregat (ES); Cebolla Gil, Ana, 08950 Esplugues de Llobregat (ES)
(74) Representative: Galenicum Health S.L.U.

(57) **Abstract**

The present invention provides pharmaceutical compositions comprising tafamidis free acid as the active pharmaceutical ingredient and pharmaceutical dosage forms containing such compositions. These formulations are intended for the treatment and prevention of polyneuropathies, particularly familial amyloid polyneuropathy (FAP) and transthyretin amyloid polyneuropathy (ATTR-PN). The invention also includes processes for the preparation of these dosage forms and methods of treating patients in need of such therapies.

## Description

### Technical Field of the Invention

The present invention relates to pharmaceutical compositions comprising tafamidis free acid as an active pharmaceutical ingredient. Furthermore, the present invention relates to a pharmaceutical dosage form comprising such a pharmaceutical composition. In addition, the invention relates to a process for the preparation of such a pharmaceutical dosage form. Additionally, the invention relates to the use of the pharmaceutical composition and of the pharmaceutical dosage form in the treatment and/or prevention of selected diseases and medical conditions, in particular polyneuropathies, including familial amyloid polyneuropathy (FAP) and transthyretin amyloid polyneuropathy (ATTR-PN). Furthermore, the present invention relates to methods of treating and/or preventing such diseases and medical conditions, wherein a pharmaceutical composition or pharmaceutical dosage form according to the invention is administered to a patient in need thereof.

### Background of the Invention

Polyneuropathies, particularly familial amyloid polyneuropathy (FAP) and transthyretin amyloid polyneuropathy (ATTR-PN), are progressive and debilitating diseases characterized by the accumulation of misfolded transthyretin (TTR) protein, which forms amyloid fibrils that deposit in peripheral nerves and other organs. These deposits lead to a gradual loss of nerve function, resulting in sensory, motor, and autonomic dysfunctions. Left untreated, these conditions can lead to severe disability, impacting quality of life, and in many cases, they are life-threatening.

Transthyretin amyloidosis, whether inherited or acquired, has historically been challenging to treat due to the progressive nature of amyloid deposition. Conventional treatment approaches, such as liver transplantation, have been used to slow the progression of the disease in some genetic cases, but they come with significant risks and limitations. Until the recent development of pharmacological agents like tafamidis, disclosed in WO 2004/056315, which stabilize the transthyretin tetramer and prevent amyloid formation, there were limited therapeutic options for patients suffering from these polyneuropathies.

Tafamidis has shown promise in delaying disease progression and improving patient outcomes, particularly by reducing the deposition of amyloid fibrils. However, the need remains for optimized pharmaceutical compositions and dosage forms that enhance bioavailability and patient compliance, while also addressing different stages of disease progression. This invention aims to address these needs by providing improved pharmaceutical formulations for the treatment of polyneuropathies using tafamidis.

Despite tafamidis' therapeutic potential, one challenge in developing pharmaceutical compositions is its polymorphic instability. Tafamidis free acid exists in different crystalline forms, including the well-known Form I disclosed in WO 2016/038500, and it is prone to polymorphic transitions under certain conditions. Such transitions can lead to variability in bioavailability, stability, and manufacturability, posing a significant challenge for pharmaceutical formulation. While there are cocrystals and other forms known from WO 2024/132369, there appears to be a need for stable compositions comprising tafamidis that ensure consistency and effectiveness throughout the product's shelf life. Developing such stable formulations is critical to ensuring the drug's efficacy and safety in clinical use.

For a generic medicine to gain regulatory approval, it must demonstrate bioequivalence to a reference product that is already on the market. This means that the generic formulation must exhibit comparable bioavailability, ensuring that the active ingredient reaches the bloodstream at a similar rate and extent as the reference product. Regulatory agencies, such as the European Medicines Agency (EMA), require that bioequivalence studies be conducted to prove that the generic product performs similarly in terms of efficacy and safety to the original product. In the case of tafamidis, the generic formulation must match the bioavailability profile of the reference product, Vyndaqel^{®} 61 mg, in order to ensure therapeutic equivalence for patients.

Different crystalline forms of a pharmaceutical compound can exhibit varying solubility properties, which directly impact their bioavailability. In the case of tafamidis free acid, crystalline forms other than Form I may demonstrate solubility profiles that differ significantly from Form I, and as such, their bioavailability should not be assumed to be comparable or self-evident. Careful consideration of these differences is necessary to ensure that any alternative crystalline form achieves the desired therapeutic effect through appropriate formulation and bioavailability studies.

WO 2020/232325 discloses the so-called Tafamidis free acid crystalline form V and its process of manufacture. US 2023/0149366 discloses solid dosage forms of tafamidis free acid.

### Aim of the Invention

The aim of the present invention is to provide a solid pharmaceutical composition comprising crystalline tafamidis free acid other than crystalline form I or other than tafamidis free acid co-crystals which exhibits the necessary technological properties for large-scale industrial manufacturing, including uniform fill weight, capsule integrity, stability, disintegration, bioavailability and dissolution. The pharmaceutical composition is designed to achieve equivalent bioavailability to the marketed reference product for tafamidis 61 mg, ensuring therapeutic equivalence and patient efficacy.

Another aim of the present invention is to provide a pharmaceutical composition comprising tafamidis free acid in the form of soft gelatin capsules which is stable, thus addressing the challenges related to polymorphic instability of tafamidis free acid.

Another aim of the present invention is to provide a pharmaceutical composition comprising tafamidis free acid that enhances the stability of the active ingredient and ensures consistent bioavailability throughout the shelf life of the product.

Another aim of the invention is to provide a pharmaceutical dosage form, specifically soft gelatin capsules, comprising tafamidis free acid, which has a rapid disintegration time, good dissolution properties, and adequate bioavailability in patients suffering from polyneuropathies, particularly transthyretin amyloidosis.

Another aim of the invention is to provide a pharmaceutical composition comprising tafamidis free acid that maintains high content uniformity and allows for efficient large-scale production, optimizing time and cost in the manufacturing process.

Another aim of the present invention is to provide a pharmaceutical composition which is bioequivalent to the commercialized product Vyndaqel^{®} 61 mg but using a polymorph other than Form I or co-crystals, ensuring therapeutic equivalence and comparable pharmacokinetic profiles.

A further aim of the present invention is to provide a process for the preparation of soft gelatin capsules containing tafamidis free acid, which is cost-effective and ensures stability during storage and handling, without compromising the efficacy of the active ingredient.

Another aim of the invention is to provide a pharmaceutical composition and dosage form, each comprising tafamidis free acid, for the prevention, slowing, or treatment of polyneuropathies, including familial amyloid polyneuropathy (FAP) and transthyretin amyloid polyneuropathy (ATTR-PN).

### Definitions

The term "active ingredient" of a pharmaceutical composition according to the present invention means the tafamidis free acid in a crystalline form other than Form I or than a cocrystal, as described herein. An "active ingredient" is also sometimes referred to herein as an "active substance."

The term "AUC" refers to Area Under the Curve in a pharmacokinetic context. AUC represents the total exposure of the body to the active ingredient, such as tafamidis free acid, after administration. It is determined by plotting the concentration of the drug in the bloodstream over time and calculating the area under the resulting curve. AUC is typically expressed in units of concentration-time (e.g., µg·h/mL) and is a key parameter for assessing the extent of drug absorption. The larger the AUC, the greater the exposure of the body to the drug. AUC is commonly used to compare bioavailability between different formulations, dosages, or conditions (e.g., fasted vs. fed state). It can be calculated over specific time intervals, such as AUC₀₋₇₂h or AUC₀₋₁₆₈h, to assess drug absorption at different phases post-administration.

The term "bioavailability" refers to the proportion of an active ingredient, such as tafamidis free acid, that enters the systemic circulation when administered and becomes available at the site of action. Bioavailability is typically expressed as a percentage and is influenced by factors such as the formulation of the pharmaceutical composition, the route of administration, and the physicochemical properties of the active ingredient. In the case of oral dosage forms, bioavailability also depends on absorption from the gastrointestinal tract. Ensuring bioavailability comparable to that of the reference product (i.e. bioequivalence) is essential for achieving the desired therapeutic effect.

The term "capsule content" refers to the pharmaceutical composition contained within a soft gelatin or hard capsule. In the case of a soft gelatin capsule, the content may consist of a liquid, semi-solid, or suspension formulation of the active ingredient, such as tafamidis free acid, along with pharmaceutically acceptable excipients. These excipients may include, among others, solvents, solubilizers, stabilizers, and other agents that enhance the stability, solubility, and bioavailability of the active ingredient. The capsule content is specifically formulated to ensure proper release and absorption of the active ingredient when the capsule is ingested by the patient.

The term "capsule shell" refers to the outer covering or casing that encloses the capsule content in a soft or hard capsule. In the case of soft gelatin capsules, the shell is primarily composed of gelatin, plasticizers (such as glycerin or sorbitol), and water, which give it flexibility and softness. The capsule shell is designed to protect the active ingredient, such as tafamidis free acid, from environmental factors like moisture and oxygen, and to facilitate the easy swallowing and release of the capsule content. The shell may also contain additional ingredients such as colorants, preservatives, or opacifiers to enhance its appearance and stability.

The term "Cmax" refers to the maximum (peak) concentration of an active ingredient, such as tafamidis free acid, in the bloodstream after administration of a pharmaceutical composition. It represents the highest observed concentration of the drug at a specific point in time, typically following absorption and prior to the start of drug elimination. Cmax is an important pharmacokinetic parameter used to assess the rate of absorption and bioavailability of the drug. The value of Cmax is typically expressed in units such as µg/mL and is often used in conjunction with other parameters, such as AUC and Tmax, to evaluate the overall pharmacokinetic profile of the drug.

The term "crystalline form IV" or "form IV" is a solid crystalline form of tafamidis free acid characterized by X-ray diffraction (XRD) peaks at 15.9, 16.9, 18.0, 27.3 ± 0.2 degrees 2-theta. The method of manufacture and characterization of this form IV of tafamidis is described in WO2016/038500 which is incorporated herein by reference in its entirety.

The term "crystalline form V" or "form V" is a solid crystalline form of tafamidis free acid characterized by X-ray diffraction (XRD) peaks at 6.0, 14.1, 17.8, 19.9, 20.5, 23.9, 25.7, 27.3, 29.1, and 31.0° ± 0.2 degrees 2-theta. The method of manufacture and characterization of this form V is described in WO 2020/232325, which is incorporated herein by reference in its entirety.

The term "co-crystal" refers to a crystalline structure composed of the active ingredient, tafamidis free acid, in association with one or more co-formers, which are typically neutral molecules. In a co-crystal, the active ingredient and co-former are present in a defined stoichiometric ratio, held together by non-covalent interactions such as hydrogen bonds, van der Waals forces, or ionic interactions. Co-crystals are distinct from salts or polymorphs of the active ingredient and may exhibit different physicochemical properties, such as solubility or stability, from those of the pure active ingredient.

The term "fed state" refers to the physiological condition of a patient after the ingestion of food. In pharmacokinetics, the fed state can significantly affect the absorption, distribution, metabolism, and excretion of an active ingredient, such as tafamidis free acid. To standardize pharmacokinetic studies, the FDA recommends the use of a high-fat, high-calorie meal to represent the fed state. This meal typically consists of 800 to 1,000 calories, with approximately 50% of the calories from fat, 25% from carbohydrates, and 15% from protein. A typical meal might include 2 eggs fried in butter, 2 slices of toast with butter, 4 ounces of hash browns, 8 ounces of whole milk, and 4 ounces of hamburger meat. This standardized diet ensures consistent evaluation of drug performance under fed conditions, where food can alter the bioavailability due to changes in gastrointestinal pH, bile secretion, and other factors.

The term "particle size distribution" refers to the range and relative proportions of particle sizes present in a pharmaceutical composition, including the active ingredient, such as tafamidis free acid, and any excipients. Particle size distribution is typically measured using techniques such as laser diffraction, microscopy, or sieve analysis, and is often reported as a statistical distribution (e.g., d10, d50, d90), where d50 represents the median particle size. Controlling the particle size distribution is crucial for ensuring uniformity in dissolution rates, bioavailability, and overall product performance. In pharmaceutical formulations, optimizing particle size distribution can influence drug absorption, stability, and manufacturing reproducibility. Unless herein indicated, the particle size measurements are performed by laser diffraction.

The term "pharmaceutical composition" refers to a dosage form that includes one or more active ingredients, such as tafamidis free acid in a crystalline form other than Form I or a cocrystal, combined with pharmaceutically acceptable excipients. The pharmaceutical composition can be solid, semi-solid, liquid, gel, suspension, or emulsion, depending on the desired formulation and delivery system. Preferably, the pharmaceutical composition is a solid pharmaceutical composition, such as a tablet, capsule, powder, or granule. In a most preferred embodiment, the pharmaceutical composition is a soft gelatine capsule. These excipients may include, but are not limited to, diluents, binders, disintegrants, solubilizers, stabilizers, and other substances that facilitate the manufacturing, stability, and bioavailability of the active ingredient. The pharmaceutical composition is designed to be suitable for administration to a patient in need, ensuring that the active ingredient is delivered in a safe, effective, and controlled manner for therapeutic purposes.

The term "Powder X-ray diffraction pattern", or "PXRD" or "X-Ray diffraction" refers to the characteristic pattern of peaks produced when a crystalline material, such as tafamidis free acid, is exposed to X-rays. As the X-rays interact with the crystal lattice, they are diffracted in specific directions based on the atomic arrangement within the crystal. The resulting pattern is unique to the crystalline structure of the material and is typically represented by a plot of intensity versus the diffraction angle (2-theta). X-ray diffraction (XRD) patterns are used to identify and characterize different crystalline forms, polymorphs, or co-crystals of a substance. The positions and intensities of the peaks in an XRD pattern provide valuable information about the crystal structure, unit cell dimensions, and degree of crystallinity. Unless herein indicated, the method used is measured with Philips X'Pert PRO X-ray powder diffractometer, equipped with Cu irradiation source =1.54184 A (Angstrom), X'Celerator (2.022° 2Θ) detector. Scanning parameters: angle range: 3-40 deg., step size 0.0167, time per step 37 s, continuous scan.

The term "ratio" refers to the quantitative relationship between two or more components in a pharmaceutical composition, expressed as the proportion of one component relative to another. Ratios are used to describe the relative amounts of different ingredients, such as active ingredients, excipients, or other formulation components. For example, in a composition containing medium chain triglycerides (MCT) and mineral oil, a ratio of 1:1 indicates equal parts of both components by weight or volume. The ratio is typically specified to ensure the desired balance of properties such as solubility, stability, and bioavailability.

The term "soft gelatin capsule" refers to an immediate-release solid dosage form in which the active ingredient, such as tafamidis free acid, is enclosed in the capsule content within a flexible, gelatin-based shell. The capsule shell typically comprises excipients such as gelatin, plasticizers (such as glycerin, sorbitol, propylene glycol, polyethylene glycol (PEG), triacetin, dibutyl sebacate, castor oil, acetylated monoglycerides, or triethyl citrate), and water. The excipients provide softness and flexibility to the capsule. Soft gelatin capsules are designed to contain either liquid, semi-solid, or suspension-based pharmaceutical compositions, which enhance the solubility and bioavailability of certain active ingredients. This dosage form is advantageous for improving patient compliance, providing ease of swallowing, and delivering the active ingredient in a controlled and stable manner.

The term "stable" as used herein refers to a pharmaceutical composition comprising tafamidis free acid wherein the content of total impurities is below 5 % (w/w), preferably 3 % (w/w), more preferably 2 % (w/w), and most preferably 1 % (w/w), as determined by liquid chromatography (HPLC) at 308 nm if such a composition is stored for 6 months at 40°C and 75 % relative humidity (RH) or 6 months at 25 °C and 60 % relative humidity (RH).

The term "tafamidis free acid" refers to the pure, free acid form of 2-(3,5-Dichlorophenyl)-1,3-Benzoxazole-6-Carboxylic Acid.

The term "Tmax" refers to the time to reach the maximum concentration (Cmax) of an active ingredient, such as tafamidis free acid, in the bloodstream after administration. It represents the time point at which the drug reaches its peak concentration, following absorption, before the elimination process begins to lower its concentration in the body. Tmax is typically expressed in hours and is used as a pharmacokinetic parameter to assess the rate of drug absorption. A shorter Tmax indicates faster absorption, while a longer Tmax suggests slower absorption. Tmax is often evaluated in conjunction with Cmax and AUC to provide a complete understanding of a drug's absorption profile.

### Summary of the Invention

In a first aspect, the present invention provides a solid pharmaceutical composition comprising crystalline tafamidis free acid, wherein said composition is free of crystalline form I of tafamidis free acid and free of tafamidis free acid co-crystals, wherein said pharmaceutical composition has an AUC₀₋₁₆₈ (h · µg/mL) 170.39 ± 18.980 when administered orally to a human in fed state at a dose of 61 mg.

In second aspect, the present invention provides a process for the preparation of a pharmaceutical composition comprising tafamidis free acid in a crystalline form other than Form I or a cocrystal, ensuring that the formulation remains stable throughout manufacturing, storage, and administration.

In a third aspect of the invention, there is provided the use of a pharmaceutical composition or a pharmaceutical dosage form of the present invention according to the present invention for the manufacture of a medicament for a therapeutic and preventive method as described hereinbefore and hereinafter.

### Detailed description of the Invention

Accordingly, the present invention provides in a first aspect a solid pharmaceutical composition comprising tafamidis free acid, wherein said composition is free of crystalline form Form I and free of tafamidis free acid co-crystals, wherein said pharmaceutical composition has an AUC₀₋₁₆₈h of 170.39 µg·h/mL ± 18.980 µg·h/mL when administered orally to an human in a fed state at a dose of 61 mg.

In a further embodiment, the present invention provides a pharmaceutical composition comprising tafamidis free acid as the active pharmaceutical ingredient in a crystalline form other than Form I or a cocrystal, and one or more excipients, particularly suitable for incorporation into soft gelatin capsules. The composition may include one or more solubilizers, plasticizers, or stabilizers to ensure optimal drug release, bioavailability, industriability, scalability and stability.

In yet a further embodiment of the first aspect, the present invention provides a pharmaceutical composition that exhibits bioequivalence to the reference product, Vyndaqel^{®} 61 mg, while utilizing a crystalline form of tafamidis free acid distinct from Form I or any cocrystal, ensuring therapeutic equivalence and safety for patients.

In an embodiment of the first aspect, the invention provides a pharmaceutical dosage form, specifically a soft gelatin capsule, comprising tafamidis free acid in a crystalline form other than Form I or a cocrystal. This dosage form offers enhanced stability and bioavailability, addressing the polymorphic instability issues associated with the known crystalline forms of tafamidis.

In an embodiment, the present invention provides a solid pharmaceutical composition comprising tafamidis free acid, wherein said composition is free of crystalline form Form I and free of tafamidis free acid co-crystals, wherein said pharmaceutical composition when administered orally to a human in fed state at a dose of 61 mg exhibits:
i. an AUC₀₋₁₆₈h of 170.39 pg.h/mL ± 18.980 µg·h/mL; and/or
ii. a Tmax of 3.75 hours ± 2.25 hours; and/or
iii. a Cmax of 4.92 µg/mL ± 0.570 µg/mL; and/or
iv. an AUC₀₋₇₂h of 139.98 pg h/mL ± 15.590 µg·h/mL; and/or

In another embodiment, the present invention provides a solid pharmaceutical composition comprising tafamidis free acid, wherein said composition is free of crystalline form Form I and free of tafamidis free acid co-crystals, wherein said pharmaceutical composition when administered orally to a human in fed state at a dose of 61 mg exhibits a Cmax of 4.92 µg/mL ± 0.57 µg/mL.

In another embodiment, the present invention provides a solid pharmaceutical composition comprising tafamidis free acid, wherein said composition is free of crystalline form Form I and free of tafamidis free acid co-crystals, wherein said pharmaceutical composition when administered orally to a human in fed state at a dose of 61 mg exhibits an AUC₀₋₇₂h of 139.98 µg·h/mL ± 15.59 µg·h/mL.

In another embodiment, the present invention provides a solid pharmaceutical composition comprising tafamidis free acid, wherein said composition is free of crystalline form Form I and free of tafamidis free acid co-crystals, wherein said pharmaceutical composition when administered orally to a human in fed state at a dose of 61 mg exhibits an AUC₀₋₁₆₈h of 170.39 µg·h/mL ± 18.98 µg·h/mL.

In another embodiment, the present invention provides a solid pharmaceutical composition comprising tafamidis free acid, wherein said composition is free of crystalline form Form I and free of tafamidis free acid co-crystals, wherein said pharmaceutical composition when administered orally to a human in fed state at a dose of 61 mg exhibits a Tmax of 3.75 hours ± 2.25 hours.

In another embodiment, the present invention provides a solid pharmaceutical composition comprising tafamidis free acid, wherein said composition is free of crystalline form Form I and free of tafamidis free acid co-crystals, wherein said pharmaceutical composition when administered orally to a human in fed state at a dose of 61 mg exhibits:
i. a Cmax of 4.92 µg/mL ± 0.57 µg/mL; and
ii. an AUC₀₋₇₂h of 139.98 pg h/mL ± 15.59 µg·h/mL.

In another embodiment, the present invention provides a solid pharmaceutical composition comprising tafamidis free acid, wherein said composition is free of crystalline form Form I and free of tafamidis free acid co-crystals, wherein said pharmaceutical composition when administered orally to a human in fed state at a dose of 61 mg exhibits:
i. a Cmax of 4.92 µg/mL ± 0.57 µg/mL; and
ii. an AUC₀₋₁₆₈h of 170.39 pg h/mL ± 18.98 µg·h/mL.

In another embodiment, the present invention provides a solid pharmaceutical composition comprising tafamidis free acid, wherein said composition is free of crystalline form Form I and free of tafamidis free acid co-crystals, wherein said pharmaceutical composition when administered orally to a human in fed state at a dose of 61 mg exhibits:
i. a Cmax of 4.92 µg/mL ± 0.57 µg/mL;
ii. an AUC₀₋₇₂h of 139.98 pg h/mL ± 15.59 µg·h/mL; and
iii. a Tmax of 3.75 hours ± 2.25 hours.

In another embodiment, the present invention provides a solid pharmaceutical composition comprising tafamidis free acid, wherein said composition is free of crystalline form Form I and free of tafamidis free acid co-crystals, wherein said pharmaceutical composition when administered orally to a human in fed state at a dose of 61 mg exhibits:
i. a Cmax of 4.92 µg/mL ± 0.57 µg/mL;
ii. an AUC₀₋₁₆₈h of 170.39 pg h/mL ± 18.98 µg·h/mL; and
iii. a Tmax of 3.75 hours ± 2.25 hours.

In another embodiment, the present invention provides a solid pharmaceutical composition comprising tafamidis free acid, wherein said composition is free of crystalline form Form I and free of tafamidis free acid co-crystals, wherein said pharmaceutical composition when administered orally to a human in fed state at a dose of 61 mg exhibits:
i. a Cmax of 4.92 µg/mL ± 0.570 µg/mL; and
ii. an AUC₀₋₇₂h of 139.98 pg h/mL ± 15.590 µg·h/mL; and
iii. an AUC₀₋₁₆₈h of 170.39 pg h/mL ± 18.980 µg·h/mL; and
iv. a Tmax of 3.75 hours ± 2.25 hours.

In a further embodiment, the solid pharmaceutical composition exhibiting all the above pharmacokinetic parameters, including Cmax, AUC₀₋₇₂h, AUC₀₋₁₆₈h, and Tmax, comprises crystalline tafamidis free acid characterized by X-ray diffraction (XRD) peaks at 6.0, 14.1, 17.8, 19.9, 20.5, 23.9, 25.7, 27.3, 29.1, and 31.0° ± 0.2 degrees 2-theta and wherein said crystalline tafamidis free acid is free of crystalline form I.

In a further embodiment, the solid pharmaceutical composition exhibiting all the above pharmacokinetic parameters, including Cmax, AUC₀₋₇₂h, AUC₀₋₁₆₈h, and Tmax, comprises crystalline tafamidis free acid from IV free of crystalline form I.

It should be clarified that neither suprabioavailability nor infrabioavailability is desired for the pharmaceutical composition of the present invention. Such variations in bioavailability may occur with certain crystalline forms of tafamidis free acid that are not encompassed by this invention. The present invention is specifically directed to a crystalline form of tafamidis free acid, other than Form I or co-crystals, which ensures bioavailability comparable to the reference product without significant deviations in absorption or therapeutic efficacy.

In a preferred embodiment, the pharmaceutical composition comprises tafamidis free acid in a crystalline form, wherein the D90 particle size of the active ingredient is controlled to be between 5 and 100 µm when measured by laser diffraction. Controlling the particle size distribution to this range ensures that the solubility, dissolution rate, and bioavailability of tafamidis free acid are optimized for oral administration in soft gelatin capsules. A D90 particle size within this range provides an ideal balance between stability and dissolution properties, enhancing the absorption of tafamidis in the gastrointestinal tract while maintaining uniformity across batches.

In a further embodiment, the tafamidis free acid crystalline form has a D90 particle size between 5 and 50 µm, as measured by laser diffraction. Reducing the particle size to this range further enhances the dissolution rate of tafamidis free acid, leading to improved bioavailability and faster onset of action when administered in a soft gelatin capsule. This smaller particle size range is particularly advantageous for formulations targeting rapid and consistent absorption of the active ingredient, without compromising the stability of the composition.

In a more specific embodiment, the tafamidis free acid crystalline form has a D90 particle size between 5 and 25 µm, as measured by laser diffraction. Achieving a particle size within this finer range provides even greater control over the dissolution profile, leading to enhanced solubility and bioavailability. This particle size distribution is ideal for ensuring that the active ingredient is rapidly and consistently absorbed in the fed state, making the formulation suitable for patients requiring immediate therapeutic action. Additionally, the controlled particle size minimizes variability in drug release and absorption across different batches of the pharmaceutical composition.

In an embodiment of the present invention the solid pharmaceutical composition is a soft gelatine capsule, a tablet or a liquid filled hard capsules. Preferably, the pharmaceutical composition is a soft gelatine capsule.

In the following, suitable excipients and carriers to be used in the pharmaceutical compositions according to the invention are described in further detail. In particular, such excipients may be used when the pharmaceutical formulation is a soft gelatine capsule.

### Capsule Fill

The following section will describe the embodiments that specifically pertain to the fill content of the soft gelatin capsule. These embodiments detail the composition of the active ingredient, excipients, and other components that make up the internal contents of the capsule, ensuring optimal solubility, stability, and bioavailability of tafamidis free acid. Each embodiment is designed to address the specific formulation needs to support the therapeutic efficacy of the pharmaceutical composition, as well as its manufacturability and performance.

In one embodiment, the pharmaceutical composition according to the present invention comprises one or more solubilizers, one or more stabilizers, one or more emulsifying agents, a vehicle, and optionally, one or more plasticizers and antioxidants. Some of the excipients may serve multiple functions simultaneously, such as acting as both a solubilizer and an emulsifier, or as a plasticizer and a stabilizer, depending on the formulation requirements of the soft gelatin capsule.

Suitable emulsifying agents, suitable solubilizers and suitable stabilizers according to the invention include, for example, lecithin, sorbitan esters such as sorbitan monolaurate, polyoxyl 35 castor oil, polyoxyl 40 hydrogenated castor oil, glyceryl monostearate, polyglyceryl esters, sucrose esters, sodium lauryl sulfate, and stearyl alcohol. These emulsifying agents facilitate the formation and stabilization of the emulsion within the soft gelatin capsule, ensuring proper dispersion of the active ingredient, tafamidis free acid. Preferred emulsifying agents include lecithin.

Suitable vehicles according to the invention include, for example, vegetable oils such as soybean oil, sunflower oil, sesame oil, medium-chain triglycerides (MCT oil), propylene glycol, glycerin, mineral oil (also referred to as liquid paraffin), caprylic/capric triglycerides, ethyl oleate, isopropyl myristate, and oleic acid. These vehicles are selected based on their ability to enhance the solubility and stability of the active ingredient, tafamidis free acid, in soft gelatin capsule formulations. Preferred vehicles include medium-chain triglycerides (MCT oil) and liquid paraffin and mixtures thereof.

Suitable antioxidants according to the invention are tocopherols (vitamin E), butylated hydroxytoluene (BHT), or ascorbyl palmitate, or mixtures thereof. The presence of an antioxidant helps to prevent oxidative reactions, ensuring the long-term stability and integrity of the soft gelatin capsule formulation. BHT is preferred.

In a preferred embodiment, the pharmaceutical composition comprises medium-chain triglycerides (MCT) with a Caprylic/Capric Triglyceride ratio of 55:45 as the vehicle. This specific blend is selected for its optimal solubilizing properties, enhancing the bioavailability of tafamidis free acid in the soft gelatin capsule formulation. The combination of Caprylic and Capric triglycerides provides an ideal balance between solubility and stability, making it particularly suitable for encapsulating lipophilic active ingredients. Additionally, the use of this MCT blend helps to improve the consistency of drug release and absorption when administered in a fed state.

In a further preferred embodiment, the pharmaceutical composition comprises a mixture of medium-chain triglycerides (MCT) with a Caprylic/Capric Triglyceride ratio of 55:45, along with liquid paraffin (mineral oil) as vehicles. This combination enhances the solubility and stability of tafamidis free acid in the soft gelatin capsule formulation. The Caprylic/Capric Triglyceride blend is selected for its excellent solubilizing properties, while liquid paraffin helps to improve the overall consistency of the formulation and provides additional stability, particularly in preventing oxidative degradation. Together, these vehicles support consistent drug release, bioavailability, and therapeutic efficacy when administered in a fed state.

Lecithin, or other suitable emulsifying agents, may interact with medium-chain triglycerides (MCTs) within the pharmaceutical composition, potentially leading to oxidative degradation of the formulation over time. Such interactions can compromise the stability and efficacy of the active ingredient, tafamidis free acid. Therefore, it is recommended to include an antioxidant.

In a further embodiment, the pharmaceutical composition includes liquid paraffin (mineral oil), medium chain triglycerides (MCT), or a mixture thereof in a range between 5% to 85% (w/w). The presence of liquid paraffin ensures a consistent viscosity and enhances the stability of the formulation, while the MCT facilitates the solubilization of the active ingredient, tafamidis free acid. The combination of these excipients helps to control the release profile and ensures consistent absorption when administered orally in a soft gelatin capsule.

In a preferred embodiment, the pharmaceutical composition comprises a combination of liquid paraffin and medium chain triglycerides (MCT) as the primary vehicles. The capsule content is formulated such that the total concentration of mineral oil, MCT, or a mixture thereof is present in a range of 5% to 85% (w/w) of the total fill weight. This range ensures optimal solubility and stability of tafamidis free acid, while also maintaining the integrity and performance of the soft gelatin capsule. The MCT and mineral oil provide a hydrophobic environment that supports the bioavailability of lipophilic active ingredients like tafamidis.

In one embodiment, the ratio of medium chain triglycerides (MCT) to mineral oil (liquid paraffin) in the pharmaceutical composition is between 0.8 and 1.6, preferably between 1.0 and 1.4, and more preferably at a ratio of 1.2. This specific ratio of MCT to mineral oil has been found to provide an optimal balance between solubilization of the active ingredient and maintaining the mechanical integrity of the soft gelatin capsule. The ratio ensures consistent drug release while enhancing the bioavailability of tafamidis free acid when administered to patients.

In a preferred embodiment, the pharmaceutical composition further comprises lecithin as an emulsifying agent, present in a concentration range of 0.5% to 10% (w/w) of the total capsule content. Lecithin helps to stabilize the emulsion formed by the MCT and mineral oil mixture, ensuring uniform dispersion of tafamidis free acid within the capsule. This range is selected to provide sufficient emulsifying action without negatively affecting the capsule's release profile or bioavailability.

In another embodiment, the ratio of medium chain triglycerides (MCT) to lecithin is maintained between 7.5 and 3.5, more preferably between 7.0 and 4.0, and even more preferably at a ratio of 6.5. This specific ratio ensures that lecithin effectively emulsifies the MCT, leading to a stable and homogenous capsule content. The MCT-to-lecithin ratio plays a critical role in ensuring the solubility and stability of the active ingredient, as well as enhancing the bioavailability of tafamidis free acid upon administration.

### Capsule shell

The shell of the soft gelatin capsule according to the invention generally comprises gelatin, a plastifying agent and water therein as the main components. Accordingly, the soft gelatin capsule can be normally kept in satisfactory conditions, so far as the water content of the shell is maintained within a certain range. The water content is preferably maintained in the range from about 5 to about 10 % for shell of a soft capsule, more preferably from about 5 to about 8% for shell of a soft capsule. However, the water content of the soft capsule is apt to vary depending upon the circumferential conditions to deviate from the above-mentioned preferred range in the-course of lapse oftime. If the water content reaches to so high level as to exceed the upper limit of the preferred range, the shell becomes wet to soften. Moreover, the soft capsules are apt to stick to each other to form an aggregated mass. On the other hand, if the water content reaches to a low level below the above-mentioned lower limit, the shell hardens to produce cracks therein. The soft capsule under these conditions shows poor lubricity and glidability so that the operations for packing the capsules cannot be carried out smoothly.

Suitable excipients for the capsule shell according to the invention include, for example, sorbitan (1,4-Sorbitan) in concentrations ranging from 6.9% to 16.0%, glycerin (also known as glycerol) in concentrations ranging from 41.0% to 45.0%, mannitol in concentrations not exceeding 4.0%, and sorbitol in concentrations ranging from 21.0% to 29.0%, with the total amount of mannitol and sorbitol not exceeding 29.0%. Water is also a crucial component, typically present in concentrations ranging from 12.8% to 14.2%. These components form the gelatin-based shell that encapsulates the pharmaceutical composition, providing flexibility and durability. Other suitable excipients for the capsule shell may include gelatin itself, polyvinyl alcohol, hydroxypropyl methylcellulose (HPMC), polyethylene glycol (PEG), and plasticizers like propylene glycol or triacetin, depending on the desired properties of the shell. Preferably, the capsule shell comprises glycerol.

In a further embodiment of the pharmaceutical composition as herein disclosed, the gelatin is an acid gelatin having from 75 to 145 g Bloom of gel strength, preferably from 85 to 135 g Bloom of gel strength, more preferably from 95 to 125 g Bloom of gel strength.

Among the different kinds of gelatins, there are A- (or acid) type and B- (or limed/alkaline) type. This categorisation essentially goes back to the pre-treatment of the raw material which will affect the characteristics of the gelatin extracted. Typical differences are the iso-electrical point as well as the viscosity in solution.

### Capsule Properties

Gel strength and viscosity are the two most important measurements used to assess the grade and quality of a gelatin. The gel strength is determined using a texture analyser. A pipet viscometer is used to determine viscosity. Both tests are based on a standard 6.67 % test solution. Gelatin weights for standard tests are made as is, without moisture correction.

Gel strength is expressed in (gram) Bloom. Commercial gelatins may vary from low Bloom (<150) medium Bloom (150 - 220) to high Bloom (> 220) types. The test was originally developed and patented in 1925 by O. T. Bloom. The procedure for gel strength determination is summarized as follows: A water solution consisting of 6.67 % gelatin (7.50 ± 0.1g gelatin and 105.0 ± 0.2g deionized water, melted at 60-65°C) is carefully prepared in a specified 150 ml, wide-mouth, glass bottle, which is then placed in a chilled water bath and held at 10 ± 0.1°C for 17 ± 1 hours. After chilling, the rigidity of the gel is measured as the force, in grams, required to impress a standard 0.500 ± 0.001 inch diameter plunger to a depth of 4 millimetres into the surface of the gel. This weight is referred to as the gel strength, or Bloom rating, of the gelatin. The greater the force required, the higher the strength of the gel. Commercial gelatins range from 50 to 300 Bloom grams.

In practice, the viscosity of gelatin is usually measured on the very same sample used for gel strength determination. The 6.67% test solution is carefully brought to 60°C whereupon 100 ml is introduced into a calibrated capillary pipet. The efflux time, in seconds, is recorded, and later converted to millipoise based on the relationship established at time of calibration. Each individual gelatin viscosity pipet is calibrated with oils traceable to the National Institute for Standards and Technology. Molecular weight distribution appears to play a more important role in the effect on viscosity than it does on gel strength. Some gelatins of higher gel strength may have lower viscosities than gelatins of lower gel strength. The viscosity of gelatin solutions increases with increasing gelatin concentration and with decreasing temperature; viscosity is at a minimum at the isoionic point.

Gelatin is generally categorized into mesh sizes 5 to 100 mesh which translates to a U.S. STD sieve size. Gelatin that is 5 mesh would look like a piece of rice where 100 mesh would look like baking flour a fine white powder. The mesh or particle size does not affect the properties of the gelatin once it is dissolved.

In a further embodiment of the pharmaceutical composition as herein disclosed, the fill is from about 35 to about 85 % w/w of the total amount of the immediate release soft gelatin capsule and the shell is from about 15 to about 65 % w/w of the total amount of the immediate release soft gelatin capsule; preferably the fill is from about 45 to about 75 % w/w of the total amount of the immediate release soft gelatin capsule and the shell is from about 25 to about 55 % w/w of the total amount of the immediate release soft gelatin capsule; more preferably the fill is from about 55 to about 65 % w/w of the total amount of the immediate release soft gelatin capsule and the shell is from about 35 to about 45 % w/w of the total amount of the immediate release soft gelatin capsule.

In a further embodiment of the pharmaceutical composition as herein disclosed, the amount of fill is from 150 mg to 400 mg, preferably from 200 mg to 350 mg, more preferably from 235 mg to 325 mg of the total amount of the immediate release soft gelatin capsule.

In a further embodiment of the pharmaceutical composition as herein disclosed, the hardness of the immediate release soft gelatin capsule is from 5.0 to 15.0 N, preferably from 7.0 and 13.0 N, more preferably from 9.0 and 11.0N. As used herein, the term "hardness" refers to the strength needed to move 2.0 mm of the surface of a soft gelatin capsule. Durometer is one of several measures of the hardness of a material, typically used as a measure of hardness in polymers, elastomers, and rubbers (e.g. Durometer Bareiss G7394-A or similar). Durometer, like many other hardness tests, measures the depth of an indentation in the material created by a given force on a standardized presser foot. The capsules are compressed to a certain extent between a measuring detector and a slowly moving plate. Under these test conditions, an optimum strength range is specified. Strength values above or below this specified range are indicative of insufficient flexibility or softening, respectively.

### Process of manufacture

In a second aspect of the invention, a method of manufacture the pharmaceutical composition according to the first aspect of the invention is described.

Methods for preparation of soft gelatin capsules are known. See, for example, J.P. Stanley, Soft Gelatin Capsules, Ch. 13 - Part Two in: The Theory and Practice of Industrial Pharmacy, eds. L. Lachman et. al., 3rd Ed., pp. 398-412, 1986, and W.R. Ebert, Soft Elastic Gelatin Capsules: A Unique Dosage Form, Pharmaceutical Technology, Vol. 1, No. 5. Soft gelatin capsules can be prepared, among other methods, by two methods. In short, the first method is known as the plate process, where a set of molds is used. A warm sheet of gelatin is laid over a lower plate and the liquid fill is poured on it. A second sheet of gelatin is then placed on top followed by the top plate. The set is placed under pressure to form the desired capsule. In sum, the second method was invented by Robert P. Scherer in 1933 and is called the rotary-die process. In this process, soft gelatin capsules are made by continuously casting two separate ribbons of molten or flowable gelatin into two separate rotating dies of an encapsulation machine to produce soft, elastic gelatin capsules.

### Therapy

In a third aspect of the invention, there is provided a method of treating or preventing polyneuropathies, particularly familial amyloid polyneuropathy (FAP) and transthyretin amyloid polyneuropathy (ATTR-PN), in a patient in need thereof, comprising administering to the patient the pharmaceutical composition comprising tafamidis free acid of the first aspect. The pharmaceutical composition is administered in the form of a soft gelatin capsule, as described herein, which enhances the bioavailability and stability of tafamidis while using a crystalline form other than form I or co-crystals. The method is intended to slow the progression of the disease, alleviate symptoms, and improve the quality of life of patients by ensuring effective delivery of tafamidis in a controlled and consistent manner.

Further aspects and embodiments of the present invention can be found in the following numbered clauses:
1. A solid pharmaceutical composition comprising crystalline tafamidis free acid, wherein said composition is free of crystalline form I of tafamidis free acid and free of tafamidis free acid co-crystals, wherein said pharmaceutical composition has an AUC₀₋₇₂ (h·µg/mL) 139.98 ± 15.590 when administered orally to a human in fed state.
2. A solid pharmaceutical composition comprising crystalline tafamidis free acid, wherein said composition is free of crystalline form I of tafamidis free acid and free of tafamidis free acid co-crystals, wherein said pharmaceutical composition has an AUC₀₋₁₆₈ (h·µg/mL) 170.39 ± 18.980 when administered orally to a human in fed state.
3. The pharmaceutical composition according to any of the preceding clauses wherein the Tmax is 3.75 hours ± 2.25 hours.
4. The pharmaceutical composition according to any of the preceding clauses wherein the Cmax (µg/mL) is 4.920 ± 0.570.
5. The pharmaceutical composition according to any of the preceding clauses wherein the crystalline form of tafamidis free acid comprises the following XRD diffraction peaks at 6.0, 14.1, 17.8, 19.9, 20.5, 23.9, 25.7, 27.3, 29.1 and 31.0° ± 0.2 degrees 2 theta.
6. The pharmaceutical composition according to any of the preceding clauses wherein the crystalline form of tafamidis free acid consists of the crystalline form characterised by the following XRD diffraction peaks at 6.0, 14.1, 17.8, 19.9, 20.5, 23.9, 25.7, 27.3, 29.1 and 31.0° ± 0.2 degrees 2 theta.
7. The pharmaceutical composition according to any of the preceding clauses wherein the composition is a soft gelatin capsule.
8. The pharmaceutical composition according to the preceding clause wherein the capsule content comprises mineral oil, medium chain triglycerides or a mixture thereof at a range between 5% to 85% (w/w).
9. The pharmaceutical composition according to the preceding clause wherein the capsule content comprises liquid paraffin, medium chain triglycerides or a mixture thereof at a range between 5% to 85% (w/w).
10. The pharmaceutical composition according to any of the two preceding clauses wherein the ratio of medium chain triglycerides to mineral oil is between 0.8 and 1.6, preferably between 1.0 and 1.4, more preferably the ratio is 1.2.
11. The pharmaceutical composition according to any of the four preceding clauses wherein the composition further comprises lecithin at a range between 0.5% to 10% (w/w).
12. The pharmaceutical composition according to the preceding clause wherein the ratio of medium chain triglycerides to lecithin is between 7.5 and 3.5, more preferably between 7.0 and 4.0, even more preferably, the ratio is 6.5.
13. The pharmaceutical composition according to any of the six preceding clauses wherein the capsule content is free from Polyethylene Glycol.
14. The pharmaceutical composition according to any of the seven preceding clauses wherein the capsule content is free from polysorbate.
15. The pharmaceutical composition according to any of the eight preceding clauses wherein the capsule content further comprises an antioxidant at a range between 0.01 to 2% (w/w).
16. The pharmaceutical composition according to the preceding clause wherein the antioxidant is Butylhydroxytoluene.
17. The pharmaceutical composition according to any of the preceding clauses wherein the tafamidis free acid crystalline form has a D90 particle size between 5 and 100 µm when measured by laser diffraction.
18. The pharmaceutical composition according to the preceding clause wherein the tafamidis free acid crystalline form has a D90 particle size between 5 and 50 µm when measured by laser diffraction.
19. The pharmaceutical composition according to the preceding clause wherein the tafamidis free acid crystalline form has a D90 particle size between 5 and 25 µm when measured by laser diffraction.
20. The pharmaceutical composition according to any of the preceding clauses for the treatment of transthyretin amyloid disease in a human.
21. A process for the preparation the pharmaceutical composition according to any of the preceding clauses comprising the following steps:
   i. Preparing a capsule fill by mixing medium chain triglycerides, liquid paraffin, an emulsifying agent and tafamidis free acid
   ii. Optionally adding an antioxidant to the capsule fill
   iii. Encapsulating the capsule fill obtained in step i. or ii. In a soft gelatine capsule
22. A solid pharmaceutical composition comprising crystalline tafamidis free acid, wherein said composition is free of crystalline form I of tafamidis free acid and free of tafamidis free acid co-crystals, wherein the crystalline form of tafamidis free acid consists of the crystalline form characterised by the following XRD diffraction peaks at 6.0, 14.1, 17.8, 19.9, 20.5, 23.9, 25.7, 27.3, 29.1 and 31.0° ± 0.2 degrees 2 theta, wherein said solid pharmaceutical composition is a soft gelatine capsule.
23. The solid pharmaceutical composition according to the preceding clause wherein the capsule content comprises a mixture of medium chain triglycerides, liquid paraffin and an emulsifying agent.
24. The solid pharmaceutical composition according to the preceding clause wherein the emulsifying agent is lecithin.
25. The pharmaceutical composition according to any of the three preceding clauses wherein the ratio of medium chain triglycerides to liquid paraffin is between 0.8 and 1.6, preferably between 1.0 and 1.4, more preferably the ratio is 1.2.
26. The pharmaceutical composition according to any of the four preceding clauses wherein the ratio of medium chain triglycerides to lecithin is between 7.5 and 3.5, more preferably between 7.0 and 4.0, even more preferably, the ratio is 6.5.
27. The pharmaceutical composition according to any of the five preceding clauses wherein the capsule content is free from Polyethylene Glycol.
28. The pharmaceutical composition according to any of the six preceding clauses wherein the capsule content is free from polysorbate.
29. The pharmaceutical composition according to any of the seven preceding clauses wherein the capsule content further comprises an antioxidant at a range between 0.01 to 2% (w/w).
30. The pharmaceutical composition according to the preceding clause wherein the antioxidant is Butylhydroxytoluene.
31. The pharmaceutical composition according to any of the nine preceding clauses wherein the tafamidis free acid crystalline form has a D90 particle size between 5 and 100 µm when measured by laser diffraction.
32. The pharmaceutical composition according to the preceding clause wherein the tafamidis free acid crystalline form has a D90 particle size between 5 and 50 µm when measured by laser diffraction.
33. The pharmaceutical composition according to the preceding clause wherein the tafamidis free acid crystalline form has a D90 particle size between 5 and 25 µm when measured by laser diffraction.
34. The pharmaceutical composition according to any of the clauses 24-33 wherein the composition comprises lecithin at a range between 0.5% to 10% (w/w).
35. A solid pharmaceutical composition comprising crystalline tafamidis free acid, wherein said composition is free of crystalline form I of tafamidis free acid and free of tafamidis free acid co-crystals, wherein the crystalline form of tafamidis free acid consists of the crystalline form IV, wherein said solid pharmaceutical composition is a soft gelatine capsule.
36. The solid pharmaceutical composition according to the preceding clause wherein the capsule content comprises a mixture of medium chain triglycerides, liquid paraffin and an emulsifying agent.
37. The solid pharmaceutical composition according to the preceding clause wherein the emulsifying agent is lecithin.
38. The pharmaceutical composition according to any of the three preceding clauses wherein the ratio of medium chain triglycerides to liquid paraffin is between 0.8 and 1.6, preferably between 1.0 and 1.4, more preferably the ratio is 1.2.
39. The pharmaceutical composition according to any of the four preceding clauses wherein the ratio of medium chain triglycerides to lecithin is between 7.5 and 3.5, more preferably between 7.0 and 4.0, even more preferably, the ratio is 6.5.
40. The pharmaceutical composition according to any of the five preceding clauses wherein the capsule content is free from Polyethylene Glycol.
41. The pharmaceutical composition according to any of the six preceding clauses wherein the capsule content is free from polysorbate.
42. The pharmaceutical composition according to any of the seven preceding clauses wherein the capsule content further comprises an antioxidant at a range between 0.01 to 2% (w/w).
43. The pharmaceutical composition according to the preceding clause wherein the antioxidant is Butylhydroxytoluene.
44. The pharmaceutical composition according to any of the nine preceding clauses wherein the tafamidis free acid crystalline form has a D90 particle size between 5 and 100 µm when measured by laser diffraction.
45. The pharmaceutical composition according to the preceding clause wherein the tafamidis free acid crystalline form has a D90 particle size between 5 and 50 µm when measured by laser diffraction.
46. The pharmaceutical composition according to the preceding clause wherein the tafamidis free acid crystalline form has a D90 particle size between 5 and 25 µm when measured by laser diffraction.
47. The pharmaceutical composition according to any of the clauses 37-46 wherein the composition comprises lecithin at a range between 0.5% to 10% (w/w).

### Analytical Procedures

### Particle Size Distribution Measurement by Laser Diffraction

Particle Size Distribution is performed for example via light scattering or laser diffraction technique. To determine the particle size the powder is fed into a laser diffraction spectrometer for example by means of a dispersing unit. The test method is described below in detail:

| | |
|---|---|
| Equipment: | Mastersizer3000 |
| Sample Dispersing Unit: | Hydro MV |
| Dispersant Medium: | Water |
| Stirrer Speed: | 2000 rpm |
| Sonication: | No |
| Optical Model: | Mie Theory |
| Material Absorption Index: | 0.01 |
| Material Refraction Index: | 1.677 |
| Number of measurements: | 2 replicates |

Each replicate consists of the average of three consecutive measurements which were stable and reproducible. The adjust with the corresponding optical model was satisfactory.

### Dissolution Test

The standard dissolution test is described in USP31-NF26 S2, chapter 711 (dissolution). For the experiments present in this patent, the Apparatus I (Baskets) method with an agitation speed of 50 rpm was used. The medium consisted of phosphate buffer with a pH of 6.8 and at a temperature of 37°C. The volume of medium per vessel was 900 ml. The tests are usually run for as long as 60 minutes. The samples are analysed by HPLC.

### HPLC Chromatographic conditions for Assay, Related Substances and Dissolution Test

| | |
|---|---|
| Column: | C18, 75 mm x 4.6 mm, 3.5 µm or equivalent |
| Flow: | 1.5 mL/min |
| Wavelength: | 308 nm |
| Column temperature: | 40 °C |
| Auto-sampler Temperature: | 22 °C |
| Injection Volume: | 40 µL |
| Run time: | 37 min |

### EXAMPLES

The following examples corresponds to a fill to be used in the manufacture of soft gelatin capsules. The fill is encapsulated to form soft gelatin capsules with shell compositions having gelatin, plasticiser agent or agents and water as described below. The manufacturing process is also as herein disclosed.

### Example 1: Preparation soft gelatin capsule

### Capsule content:

| **Ingredients** | **Function** | **mg** |
|---|---|---|
| Tafamidis free acid | Active Ingredient | 61.00 |
| Medium Chain triglycerides | Vehicle | 130.10 |
| Liquid Paraffin | Vehicle | 108.50 |
| Lecithin | Emulsifying agent | 20.00 |
| Butylhydroxytoluene | Antioxidant | 0.035 |
| Total fill content (mg/capsule) | - | 319.6 |

### Capsule shell:

| **Ingredients** | **mg** |
|---|---|
| Gelatin A | 205.00 |
| Glycerol | 75.00 |
| Purified water | 1609.65 |
| Titanium Dioxide (E171) | 1.7 |
| Iron oxide Yellow (E172) | 0.84 |

### Example 2: Preparation soft gelatin capsule

### Capsule content:

| **Ingredients** | **Function** | **mg** |
|---|---|---|
| Tafamidis free acid | Active Ingredient | 61.00 |
| Medium Chain triglycerides | Vehicle | 153.60 |
| Liquid Paraffin | Vehicle | 85 |
| Lecithin | Emulsifying agent | 20.00 |
| Butylhydroxytoluene | Antioxidant | 0.035 |
| Total fill content (mg/capsule) | - | 319.6 |

### Capsule shell:

| **Ingredients** | **mg** |
|---|---|
| Gelatin A | 205.00 |
| Glycerol | 75.00 |
| Purified water | 1609.65 |
| Titanium Dioxide (E171) | 1.7 |
| Iron oxide Yellow (E172) | 0.84 |

### Example 3: Preparation soft gelatin capsule

### Capsule content:

| **Ingredients** | **Function** | **mg** |
|---|---|---|
| Tafamidis free acid | Active Ingredient | 61.00 |
| Medium Chain triglycerides | Vehicle | 130.1 |
| Liquid Paraffin | Vehicle | 108.50 |
| Lecithin | Emulsifying agent | 40.00 |
| Butylhydroxytoluene | Antioxidant | 0.035 |
| Total fill content (mg/capsule) | - | 319.6 |

### Capsule shell:

| **Ingredients** | **mg** |
|---|---|
| Gelatin A | 205.00 |
| Glycerol | 75.00 |
| Purified water | 1609.65 |
| Titanium Dioxide (E171) | 1.7 |
| Iron oxide Yellow (E172) | 0.84 |

### General Manufacturing Process for Tafamidis Free Acid Soft Gelatin Capsules

### 1. Preparation of the Capsule Content

1.1 Medium-chain triglycerides (MCT) and liquid paraffin were added to a stainless steel tank equipped with a cooling jacket to maintain the temperature between 32-34°C. The mixture was stirred until homogeneously dissolved.
1.2 Lecithin was then added to the tank and stirred until completely dissolved.
1.3 Butylhydroxytoluene (BHT) was added next and stirred until fully dissolved.
1.4 Finally, tafamidis free acid was added to the tank and stirred continuously until fully dissolved and uniformly distributed throughout the mixture.

### 2. Preparation of the Capsule Shell

2.1 Purified water and glycerol were weighed and transferred to a reactor.
2.2 The mixture was heated to 80°C while stirring.
2.3 Gelatin A was added to the mixture slowly, with continuous stirring at a low speed to ensure even dissolution and prevent foam formation.
2.4 Once the gelatin was completely dissolved, the titanium dioxide and iron oxide yellow colorants, previously dissolved in a small amount of purified water, were added to the mixture.
2.5 The complete mixture was stirred until a homogeneous gelatin mass was formed, suitable for the encapsulation process.

### 3. Capsulation Process

3.1 The gelatin mass was laminated into two sheets, and the thickness of each band was adjusted according to the specifications required for the capsule shell.
3.2 The prepared capsule content (tafamidis mixture) was filled into the gelatin sheets, forming the soft gelatin capsules.
3.3 The filled capsules were then transferred to a drying room where they were dried under controlled conditions to achieve the desired mechanical strength and moisture content.

### 4. Packaging

4.1 Primary packaging: The dried soft gelatin capsules were packed into blister packs with the following specifications:
4.2 20-micron aluminum lacquer sealable to PVC/PVDC.

### Example 4: Stability

| | **t=0** | **25 °C / 60% RH-6 month** | **30 °C / 65% RH - 6 month** | **40 °C / 75% RH-6 month** |
|---|---|---|---|---|
| **Appearance** | opaque and yellow soft gelatin capsule | opaque and yellow soft gelatin capsule | opaque and yellow soft gelatin capsule | opaque and yellow soft gelatin capsule |
| **Polymorph interconversion** | - | No | No | No |
| **Assay (%)** | 99.6 | 99.6 | 99.5 | 99.5 |
| **RELATED SUBSTANCES** | ND | ND | ND | ND |
| Unknown individual impurities (%) | | | | |
| **Total impurities (%)** | ND | ND | ND | ND |

| | | | | |
|---|---|---|---|---|
| ND= Not detected. Reporting threshold: 0.1%. | | | | |

### Example 5: Excipient Ratio Summary

| | Example 1 | Example 2 | Example 3 |
|---|---|---|---|
| Ratio MCT/Liquid Paraffin (Mineral oil) | 1.2 | 1.8 | 1.2 |
| Ratio MCT/Lecithin | 6.5 | 7.7 | 3.3 |

### Example 6: Summary of Crystalline forms & PSD for examples 1-3

| | **Example 1** | **Example 2** | **Example 3** |
|---|---|---|---|
| Crystalline form PXRD | 6.0, 14.1, 17.8, 19.9, 20.5, 23.9, 25.7, 27.3, 29.1 and 31.0° ± 0.2 degrees 2 theta | | |
| PSD (D90) by laser diffraction | 6 µm | | |

### Examples 7, 8 and 9: Other crystalline forms

Examples 1-3 were repeated using crystalline form IV of tafamidis. Similar stability results and dissolution profiles to the formulations of examples 1-3 were observed.

| | **Example 7** | **Example 8** | **Example 9** |
|---|---|---|---|
| Crystalline form PXRD | Form IV | | |
| PSD (D90) by laser diffraction | 6 µm | | |

### Example 10: Bioavailability (fed study) in humans

A bioavailability study was conducted with 66 healthy human adult subjects (gender: 10 female and 56 male, age range: 18-45 years). All 66 subjects completed the study, which was performed using the soft gelatin capsule formulation of tafamidis free acid of Example 1. The study followed an open-label, single-dose in healthy subjects under fed conditions. Table below reports the mean pharmacokinetic parameters, the observed standard deviation (SD) for Cmax, AUC and Tmax values.

| Parameter | Example 1 |
|---|---|
| AUC₀₋₇₂h (µg·h/mL) | 139.98 ± 15.590 |
| AUC₀₋₁₆₈h (pg.h/mL) | 170.39 ± 18.980 |
| Cmax (µg/mL) | 4.92 ± 0.570 |
| Tₘₐₓ (h) | 3.75 ± 2.25 |

Outcome: The formulation of Example 1 has been found to be bioequivalent to the commercial reference product Vyndaqel 61 mg.

### Example 11: PSD impact

With the formulations of Examples 1-3 and 7-9, the PSD D90 in the range 5-100 µm as measured by laser diffraction has shown not to impact the dissolution profiles carried according to the dissolution method disclosed herein. It is therefore reasonably expected that the bioavailability within said range is not impacted.

## Claims

1. A solid pharmaceutical composition comprising crystalline tafamidis free acid, wherein said composition is free of crystalline form I of tafamidis free acid and free of tafamidis free acid co-crystals, wherein said pharmaceutical composition has an AUC₀₋₁₆₈ (h · µg/mL) 170.39 ± 18.980 when administered orally to a human in fed state at a dose of 61 mg.

2. The pharmaceutical composition according to the preceding claim wherein the Tₘₐₓ is 3.75 hours ± 2.25 hours.

3. The pharmaceutical composition according to any of the preceding claims wherein the Cₘₐₓ (µg/mL) is 4.920 ± 0.570.

4. The pharmaceutical composition according to any of the preceding claims wherein the crystalline form of tafamidis free acid consists of the crystalline form **characterised by** the following XRD diffraction peaks at 6.0, 14.1, 17.8, 19.9, 20.5, 23.9, 25.7, 27.3, 29.1 and 31.0° ± 0.2 degrees 2 theta.

5. The pharmaceutical composition according to any of the preceding claims wherein the composition is a soft gelatin capsule.

6. The pharmaceutical composition according to the preceding claim wherein the capsule content comprises liquid paraffin, medium chain triglycerides or a mixture thereof at a range between 5% to 85% (w/w).

7. The pharmaceutical composition according to any of the two preceding claims wherein the ratio of medium chain triglycerides to liquid paraffin is between 0.8 and 1.6, preferably between 1.0 and 1.4, more preferably the ratio is 1.2.

8. The pharmaceutical composition according to any of the three preceding claims wherein the composition further comprises an emulsifying agent at a range between 0.5% to 10% (w/w).

9. The pharmaceutical composition according to the preceding claim wherein the ratio of medium chain triglycerides to emulsifying agent is between 7.5 and 3.5, more preferably between 7.0 and 4.0, even more preferably, the ratio is 6.5.

10. The pharmaceutical composition according to any of the five preceding claims wherein the capsule content is free from Polyethylene Glycol.

11. The pharmaceutical composition according to any of the six preceding claims wherein the capsule content is free from polysorbate.

12. The pharmaceutical composition according to any of the seven preceding claims wherein the capsule content further comprises an antioxidant at a range between 0.01 to 2% (w/w).

13. The pharmaceutical composition according to any of the preceding claims wherein the tafamidis free acid crystalline form has a D90 particle size between 5 and 100 µm when measured by laser diffraction.

14. The pharmaceutical composition according to any of the preceding claims for the treatment of transthyretin amyloid disease in a human.

15. A process for the preparation the pharmaceutical composition according to any of the preceding claims comprising the following steps:
i. Preparing a capsule fill by mixing medium chain triglycerides, liquid paraffin, an emulsifying agent and tafamidis free acid,
ii. Optionally adding an antioxidant to the capsule fill
iii. Encapsulating the capsule fill obtained in step i. or ii. In a soft gelatine capsule
